Europäisches Patentamt

(19) European Patent Office

Office européen des brevets

(11) Publication number: **0 344 134 B1**

(12) **EUROPEAN PATENT SPECIFICATION**

(45) Date of publication of patent specification: **19.01.94**   (51) Int. Cl.⁵: **C07K 15/00**, C12P 21/00, C12N 5/00, G01N 33/574, //C12N15/00,(C12P21/00, C12R1:91)

(21) Application number: **89830234.4**

(22) Date of filing: **25.05.89**

(54) Monoclonal antibody specific to a fibronectin sequence expressed in transformed cells, hybridoma secreting said antibody and use of said monoclonal antibody in the diagnosis of tumors.

(30) Priority: **26.05.88 IT 2074188**

(43) Date of publication of application:
**29.11.89 Bulletin  89/48**

(45) Publication of the grant of the patent:
**19.01.94 Bulletin  94/03**

(84) Designated Contracting States:
**AT BE CH DE FR GB IT LI LU NL SE**

(56) References cited:

JOURNAL CELL BIOLOGY, vol. 108, March
1989, The Rockefeller University Press; B.
CARNEMOLLA et al., pp. 1139-1148&NUM;

CHEMICAL ABSTRACTS, vol. 108, 1988, Columbus, OH (US); T. VARTIO et al., p. 478, no.
53358c&NUM;

CHEMICAL ABSTRACTS, vol. 107, 1987, Columbus, OH (US); L. BORSI et al., p. 534, no.
56711c&NUM;

(73) Proprietor: **ISTITUTO NAZIONALE PER LA
RICERCA SUL CANCRO**
**Viale Benedetto XV, 10**
**I-16132 Genova(IT)**

(72) Inventor: **Zardi, Luciano**
**Viale Benedetto XV, 10**
**I-16132 Genova(IT)**

(74) Representative: **Minoja, Fabrizio**
**Studio Consulenza Brevettuale**
**Via Rossini, 8**
**I-20122 Milano (IT)**

**Description**

The present invention relates to a specific monoclonal antibody to a protein sequence coded for by the exon ED-B of human fibronectin, to an hybridoma secreting said antibody and to the use of said antibody in the diagnosis of tumors.

Fibronectins are a class of high molecular weight glycoproteins present in soluble form in plasma and other body fluids and in insoluble form in the extracellular matrices. Fibronectin molecules are involved in various biological phenomena, such as the maintenance of normal cell morphology, cell migration, hemostasis and thrombosis, wound healing and oncogenic transformation. Fibronectin (FN) is known to consist of a mixture of molecules of different structures (isoforms) whose characteristics vary depending on the originary tissue. FN from transformed human cell - cultures has an isoform composition very different from the normal counterpart. As a matter of fact, transformed cells produce remarkable amounts of a FN isoform ($\beta$-FN) which is poorly present in normal cells. Recently we demonstrated that this FN form derives from a differential splicing pattern of the FN mRNA precursor which leads in transformed cells to a high-level expression of the ED-B exon which, on the contrary, is poorly expressed in normal cells (EMBO J., Volume 6, pages 2337-2342, 1987).

The monoclonal antibody according to the present invention allows the analysis of the isoform composition of FN in human tissues obtained from biopsies and therefore is suited for many important diagnostic and/or therapeutic uses.

The antibody of the invention, due to its high affinity and specificity, provides an ideal reagent for evidencing the oncofetal FN isoform in biopsias of human tissues.

According to a further object of the invention, an hybridoma is provided obtained by fusing spleen cells from immunized mice, using FN derived from a culture medium of SV40-transformed embryonic cells with myeloma cells from P3UI mice.

FN which had been purified from the culture medium of SV40-transformed Wi-38 embryonic human lung cells and defined Wi-38Val3, was used to immunize the animals. The used immunization and fusion techniques are the conventional ones and they will be described in detail in the Example hereinafter. The antibody-secreting hybridoma of the invention was deposited on April 21, 1988 at the European Collection of Animal Cell Cultures, Porton Down, Salisbury, Great Britain, under the n° 88042101.

The monoclonal antibody of the invention was found to be highly specific and selective for an epitope present in the ED-B sequence of FN from Wi-38Val3 cells, as it could be demonstrated using the Western Blot technique, with plasma FN or thermolysin-digested FN from Wi-38 or Wi-38Val3 cells. The monoclonal antibody of the invention does not react neither with plasma FN nor with FN from Wi-38 normal human fibroblasts.

In order to evaluate the applicative possibilities of the antibody of the invention, the distribution epitope recognized by the antibody was investigated in a variety of fetal and adult tumoral and normal tissues.

The results from the immuno-histochemical analysis of a variety of fetal and adult normal tissues of different embryonic origins using the Mab of the invention are summarized in Table I.

2

## TABLE I

Reactivity of the Mab of the invention with adult and fetal normal tissues (8-10 weeks)

| | | |
|---|---|---|
| Fetal tissues | Negative | : colon, skin, kidney |
| | Positive | : brain, stomach, thymus, lung, jejunum, liver |
| Adult tissues | Negative | : brain(2), lung(3), breast(3), stomach(4), duodenum(3), colon(3), liver(2), pancreas(1), kidney(4), urinary bladder(2), prostate(2), testis(2), endometrium(2), spleen(2), lymph nodes(2), skin(6), thymus(2), thyroid(2), meninges(2), skeletal muscle(1), choroid(2), retina(2). |
| | Positive | : synovia(3), ovary(1), Fallopian tube(1/4). |

( ) between brackets the number of individuals from which tissues were obtained is indicated.

The studies carried out show that the recognized epitope has a highly specific tissue distribution. Among 10-12 weeks old fetal tissues, the Mab of the invention colors the inner coat of the blood vessels of stomach, jejunum, thymus and lung submucosa as well as the basement membrane of bronchial epithelium (pseudo glandular stage). With the exception of the basal portion of gastric glands, the older fetal tissues (up to 26 weeks) proved to be completely negative with the Mab of the invention, thus suggesting that the FN epitope identified by this reagent is subjected to a programmed expression during ontogenesis. In the adult samples the tissue distribution of said epitope, although different from the fetal condition, is much more restricted. Mab of the invention was found to color surface synovial cells, the inner coat of some ovarian vessels, isolated areas of the basement membrane of celomic epithelium and, in a varying intensity, myometrium areas. Whereas normal tissues very seldom react with the Mab of the invention, as it is evident from Table II, the major part of the analyzed tumors contained remarkable levels of the epitope recognized by the antibody under test.

## TABLE II

| Kind of tumor | positive n./tested n. |
|---|---|
| Stomach adenocarcinoma | 3/7 |
| Pancreas " | 0/3 |
| Liver " | 2/7 |
| Colon " | 2/10 |
| Kidney " | 4/8 |
| Urinary bladder carcinoma | 4/8 |
| Prostata adenocarcinoma | 1/5 |
| Ovaries " | 2/8 |
| Endomethrium " | 1/6 |
| Skin – mixed type | 3/8 |
| Thyroid adenocarcinoma | 1/8 |
| Lung " | 7/13 |
| Skin and eyes melanomas | 9/21 |
| Breast adenocarcinoma | 3/13 |
| Brain – mixed type | 4/13 |
| Meningioma | 13/13 |
| Sarcomas of mixed type | 3/15 |

The incidence of the positive coloration varied within a given type of tumor and no relationships with the differentiation degree (colon adenocarcinomas) nor with the tumor histiotype (breast tumors) were evidenced. The only exception is represented by fibromatous and endothelial meningiomas, which were found to be systematically positive for the carcinoma cases, in which both the normal tissue and the tumoral one from the same patient could be studied, the positive reaction with the Mab of the invention was found only in the neoplastic tissue. In almost all the samples which were positive with the Mab of the invention, the coloration was located in areas of varying extension of the interstice of the tumor, which surrounded or divided tumor cell nestes of variable measures, in which the coloration seldom delimited individual cells.

A frequent aspect of the tumors which were positive with the Mab of the invention was that the inner coat of the vessels was positive, whereas the one from the tested normal adult tissues was never positive, with the exception of some ovarian blood vessels.

A certain number of benign lesions (breast fibrosadenoma (4), mastopathias (2), gynecomastias (4), prostatic hypertrophy (5), urinary bladder and rectum polyps (4), intradermal nevi (14), angiomas (2), ovarial cystoadenomas (2), thyroid neurofibromas (1), chronic inflammatory lesions, hepatic cirrhosis (2), Echinococcus cysts (2), cutaneous scars (3) (10-30 days)) were tested with the Mab of the invention and all of them were found to be negative, with the exception of one breast adenofibroma which showed a coloration of the interstitial tissue in some areas.

From what stated above it is evident that the Mab of the present invention can be useful for diagnostic purposes for an early and sensitive diagnosis of tumors. To this purpose, the Mab can be employed in known immunoenzymatic, immunofluorescence techniques and the like, in which techniques the monoclonal antibody is labelled with enzymes, fluorescent compounds, luminescent compounds, radioactive compounds, ferromagnetic probes and the like. Particularly preferred is the enzyme labelling, in which case alcaline phosphatase, glucose oxidase, galactosidase, peroxidase and urease are usually employed. Enzyme labelling can be carried out according to any conventional methods, using for example glutaraldehyde, benzoquinone, periodate, protein A etc.

Once the labelled monoclonal antibody is obtained, analysis is performed using one of the many conventional immunoassay methods, for example those known as EIA or ELISA. Those skilled in the art, when the Mab of the invention is available, could easily develop a diagnostic kit or a suited method to detect transformed FN from bioptic samples. A positive response (i.e. due to the recognized presence of FN containing the ED-B sequence) is indicative of a tumoral or viral transformation occurred in the cells of the examined tissue.

Due to the specificity of the Mab of the invention to tumor-transformed cells, the antibody itself, suitably bound to cytotoxic or anyway antitumoral medicaments can be used for therapeutic purposes in form of appropriate pharmaceutical compositions, which are a further object of the present invention.

The following non-limiting example illustrates the invention in more detail.

## EXAMPLE

### Preparation of the antigen.

The culture medium is drawn from culture confluent plates of Wi38Val3 cells (SV40-transformed fibroblasts) cultured in the presence of FCS from which bovine FN had been removed. The medium is filtered on Millipore filters, added with $NaN_3$ and trasylol (final concentration 0,2% and 10 $\mu$/ml respectively), then passed through a Sepharose 4B-conjugated gelatin chromatographic column. Up to 20 liters of medium are charged on a 150 cc column.

Thereafter the column is washed with PBS (20 mM Na phosphate pH 7; 0,15 NaCl) containing 0,1% $NaN_3$, then with 4M NaCl in 50 nM Tris, pH 7, to remove the specifically bound proteins. Finally FN is eluted with 3M urea in PBS containing 0,1% $NaN_3$.

The whole procedure is carried out at room temperature, with a 200 ml/hr flow rate, recovering 10 ml fractions.

The fractions obtained washing the column with 3M urea and having an absorbance at 200 nm higher than 0,5 are combined in a pool and dialyzed against 20 volumes of PBS containing 0,1% $NaN_3$ and 2 mM PMSF.

Usually 5 mg of FN are obtained from 1 liter of medium. The FN obtained by this procedure turns out to be homogeneous in electrophoresis on polyacrylamide gels.

### Mice immunization and cell hybridization techniques

Balb/c mice are immunized with two intraperitoneal injections of purified FN, 50 $\mu$g, in about 100 $\mu$l of PBS emulsified with an equal volume of complete Freund adjuvant (DIFCO) and with three intraperitoneal injections containing 400 $\mu$g of antigen with no adjuvant. The two first injections are applied at 1 week intervals, the subsequent ones being administered daily during the 3 last days before the fusion.

Spleens are sterile removed and broken up into a single cell suspension using a stainless steel dense mesh net. Fusion is carried out by incubating 10 mouse splenocytes with 1/5 P3UI plasmocytoma cells in 0.5 ml of polyethylene glycol (PEG 4000) with 50% DMEM-FCS, added dropwise in one minute. Then the mixture is shaken during 90 second at 37° C. After that, 20 ml of serum-free DMEM are added dropwise under stirring. The mixture is left to stand for 5 minutes; then 20 ml of DMEM containing 10% fetal calf serum (FCS) are added and the cells are centrifuged at 1000 x g for 5 min. The supernatant is removed and the cells are resuspended in 48 ml of DMEM-HAT (20% FCS, 1% hypoxanthine, 1% aminopterin, 0.2% thymidine, 0.2% glycine). The cells supension is then dispensed (1 ml per well) in two 24-well plates containing a monolayer of macrophages. 3 days after 1 ml of HAT medium is added to each well.

Said procedure is carried out for 2 weeks, at the end of which the growth of hybrids on the bottom of the 48 wells is observed.

## Cloning

The presence of anti-FN antibodies in each well is checked by radioimmunoassay, then the cells contained in the "positive" wells are cloned.

The cells are removed from each well and resuspended in 10 ml of HAT medium, counted and diluted to obtain a suspension containing about 3 cells/ml.

About 200 $\mu$l of this dilution is dispensed per well in 96 wells already containing mouse peritoneal macrophages.

About 2 weeks after, the medium from those wells having at least 1/3 surface covered with cells is assayed for anti-FN antibodies by radioimmunoassay.

## Radioimmunoassay

Radioimmunoassay is performed accordingly to the suitably modified method described by Accolla and Celada [Accolla, R.S., Celada, F. (1978) Eur. J. Immunol. 8, 688].

96-well polyvinyl plates ("Microvil" 24 A m.cat. 968 Pbi) are used. Polyvinyl is a material characterized by having binding sites for proteins. FN (at a concentration of 100 $\mu$g/ml) purified according to the above described method is incubated in the wells at room temperature. 2 hours after the protein is removed from the wells, which are filled with PBS containing 1% bovine serum albumin (200 $\mu$l per well) to take up unoccupied binding sites. After two further hours the albumin solution is removed and the wells are washed with PBS. Then the culture media of the hybrids whose capability to release anti-FN antibodies is to be checked are added (100 $\mu$l per well). After 2 hours more said media are removed off and the wells are washed again with PBS. At this point, mouse anti-FAB antibodies induced in rabbits, labelled with I are added, 100 $\mu$l per well, and incubated at room temperature for 2 hours. Subsequently after washing the unbound radioactive antibodies with PBS each well is placed into a numbered test tube. Radioimmunoassay is carried out by means of a gamma-counter (Packard) for a period of 10 minutes for each tube.

## Determination of the antibody specificity

Among the hybridomas prepared by the described method, a clone was obtained which releases antibodies which react with Wi38Val3 FN and do not react with FN from plasma or normal cells. This specificity was assayed by two different methods:

A) Radioimmunoassay.

FNs purified from Wi38Val3 cells, from normal fibroblasts or from plasma culture media were used for the radioimmunoassay, according to the above described method.

The antibody under test gave counts with positive values only with FN from Wi38Val3 cells.

B) Western blot.

FNs purified from Wi38Val3 cells, from normal fibroblasts or from human plasma culture media was caused to migrate on a SDS polyacrylamide gel according to the method by Laemmli [Laemmli, U.K. (1970) Nature, 227, 680-685].

After the electrophoresis, the proteins were transferred on a nitrocellulose filter (Western blot) according to the method described by Towbin et al. (1979), Proc. Natl. Acad. Sci., 76, 4350-4354.

After that, filters were washed with 6 M urea in distilled water for 10 minutes, to remove SDS. After thorough washing with distilled water, then with TBS (10 mM Tris-HCl pH 7,4; 0,15 M NaCl), the filters were incubated for 2 hours with anti-FN monoclonal antibodies containing 0,05% Tween 20 (Merck). The anti-FN antibodies excess was removed by washing with TBS (5 changes, 30 minutes total); then filters were incubated for 2 hours with a second antibody conjugated with mouse anti-Ig peroxidase (Dako, Copenhagen, Denmark) diluted 1/1000 in TBS, 3% BSA, 0,05% Tween 20.

After repeated washings with TBS (5 changes, 30 minutes, total) and two washings with TB (10 nM Tris-HCl pH 6,8), filters were incubated with a substrate solution to visualize the antigen bands. Substrate was prepared immediately before use, as followings:

20 ml of TB, 6,63 $\mu$l of 30% $H_2O$, 5 ml of a 0.3% (w/v) 4-chloro-1-naphthol solution (Merck; Darmstad, West Germany) in methanol.

4-Chloro-1-naphthol solution can be preserved for one month at 4°C. The reaction is quenched by washing filters with distilled water after 10-120 minutes (the time depending on the antigen amounts and on the activity of the first antibody). Filters are dried between two paper filters and photographed. By this technique, the only protein which can react with the antibody under test is FN from Wi38Val3 cells.

## Localization of the antigenic determinant recognized by the Mab

The Western blot technique was used to localize the epitope recognized by this antibody.

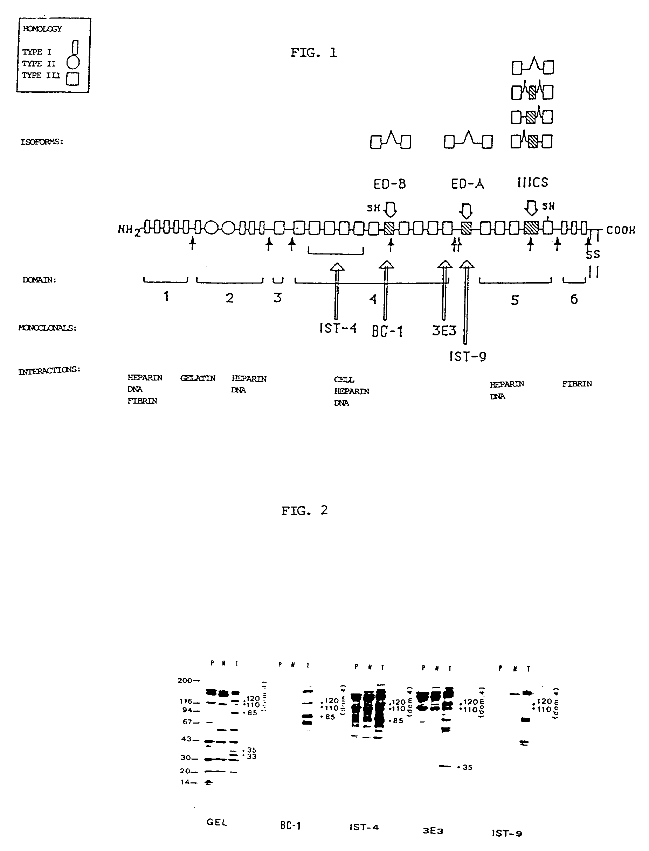

FNs from Wi38Val3 cells, from normal fibroblasts and from plasma were thermolysin digested. Each type of FN was purified, dialyzed in 25 mM Tris-HCl, pH 7,6, against 0.5 mM EDTA, 50 mM NaCl, 2.5 mM $CaCl_2$ and incubated with thermolysin (5 $\mu$m/mg FN) for 4 hours at 22°C. EDTA was added to a final 5 mM concentration to quench the reaction. The obtained digests were caused to migrate on a SDS polyacrylamide gel, according to the method by Laemmli, U.K. (1970) Nature, 227, 680-685 to separate the protein fragments depending on the molecular weight thereof. Then peptides were transferred on a nitrocellulose sheet, by the Western blot technique. Different filters were prepared with the same sample series, to test some of them with Mabs whose antigenic determinants have a known location on the FN molecule, whereas on one of these filters the Mab according to the invention was tested, which is hereinafter defined as BC-1. [Fig. 1 - Model of the domain structure of a subunit of human FN. Big white arrows indicate the regions of variability due to alternative splicing of the FN mRNA precursor. Long white arrows indicate the sites in which the epitopes recognized by the various Mabs are located. The figure also shows the three types of internal homologies, the macromolecules interacting with the various FN domains and the possible isoforms generated by different splicing models. Fig. 2 -Electrophoresis on SDS polyacrylammide 4-18% gel of plasma FN (P); FN from Wi38 normal human cells (N) and from Wi38Val3, SV40-transformed human fibroblasts (T) digested by thermolysin (5 $\mu$g/mg of FN for 2 hours at 22°C). The values on the left are the molecular masses of standards in kDa. The values on the right are the molecular masses in kDa of the four major fragments present only in the thermolysin digest of FN from transformed cells and of the 110-kDa fragment, which constitutes domain 4. Immunoblots of similar gels, respectively colored with BC-1; IST-4; 3E3 and IST-9 are also shown. Evidenced are the two forms of domain 4, plus or without the ED-B sequence.]

This antibody strongly reacts with a 120 kDa fragment which represents the cell binding form containing the ED-B sequence [Zardi, L., Carnemolla, B., Siri, A., Petersen, T.E., Paolella, G., Sebastio, G., Baralle, F.E. (1987) EMBO J., 6, 2337-2342] but does not react with the 110 kDa fragment, which represents the cell binding domain without the ED-B sequence (figure 1). This datum indicates that the epitope recognized by the BC-1 antibody is contained in the ED-B sequence.

In order to prove further this result, the two 120 and 110 kDa fragments were purified and further digested.

120 and 110 kDa fragments to FN (figure 1) were purified from a FN thermolysin digest (6 $\mu$g/mg of FN for 2 hours at 22°C) by a hydroyapatite (DNA-Grade, BioRad Laboratories, Richmond, Ca) chromatographic column as previously described [Borsi, L., Castellani, P., Balza, E., Siri, A., Pellecchia, C., De Scalzi, F., Zardi, L. (1986) Anal. Biochem., 155, 335-345]. Complete separation of the 120 kDa from the 110 kDa fragment was achieved using a DEAE-cellulose (DE+52, Whatman, Maidstone, UK) chromatographic column. Both the 120 and 110 kDa fragments were digested with thermolysin.

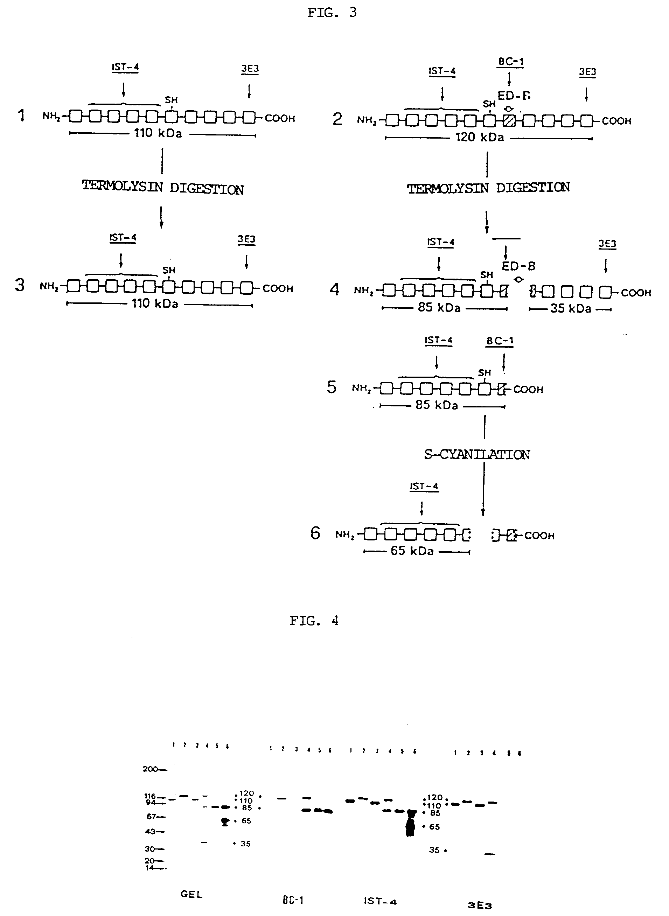

110 kDa fragment is resistant to thermolysin whereas the presence of the ED-B sequence in the 120 kDa fragment introduces a thermolysin-sensitive site in this molecule [Zardi, L., Carnemolla, B., Siri, A., Petersen, T.E., Paolella, G., Sebastio, G., Baralle, F.E. (1987) EMBO J., 6, 2337-2342]. [Figure 3 - Schematic representation of thermolysin digestion of the 110 kDa fragment (domain 4 without ED-B) and of the 120 kDa fragment (domain 4 plus the ED-B sequence). The cut by means of S-cyanilation of the 85 kDa fragment obtained in the domain 4 (120 kDa) is schematically shown. The epitopes recognized by Mabs 3E3, IST-4 and BC-1 are also indicated. The numbers on the left show the corresponding columns in the gels and immunoblots reported in figure 4. Figure 4 - On the left is reported the electrophoresis on SDS polyacrylamide gel 4-18% of the following examples: purified 110 kDa fragment (lane 1); purified 120 kDa fragment containing the ED-B sequence (lane 2); purified 110 kDa fragment digested with thermolysin (lane 3); 120 kDa fragment digested with thermolysin (lane 4); purified 85 kDa fragment from the thermolysin digest of 120 kDa fragment (lane 5) and the 85 kDa fragment cut by S-cyanilation (lane 6). On the right the immunoblots of similar gels colored using Mabs BC-1, IST-4 and 3E3, respectively, are reported.]. Thus, thermolysin digestion of domain 4 from 120 kDa gives two fragments: a 35 kDa and an 85 kDa fragment.

These fragments were tested after electrophoresis and blotting with Mab BC-1 and the obtained results proved that the only 85 kDa fragment reacts with Mab BC-1.

The determination of the amino acid sequence proved that 35 kDa fragment contains 7 amino acids of the ED-B sequence in its amino terminal portion; the 85 kDa fragment contains the ED-B sequence almost complete in its carboxy terminal portion.

The 85 kDa fragment was purified and its carboxy terminal portion was cut by S-cyanilation [Sekiguchi, K., Hakomori, S. (1983) Biochemistry, 22, 1415-1422] to produce a 65 kDa fragment which cannot any longer react with Mab BC-1 (see Figures 3 and 4).

## Claims
### Claims for the following Contracting States : AT, BE, CH, DE, FR, GB, IT, LI, LU, NL, SE

1. Monoclonal antibody specific to the ED-B sequence of fibronectin from transformed cells, obtained from the hybridoma deposited at the European Collection of Animal Cell Cultures (ECACC) on April 21, 1988, under the No. 88042101.

2. Hybridoma ECACC 88042101.

3. A method for the diagnosis of tumors in bioptic samples, characterized in that said samples are tested with the monoclonal antibody of claim 1 or with the same antibody, suitably labelled, for the presence of transformed fibronectin.

4. The method according to claim 3, which uses the monoclonal antibody labelled with enzymes, fluorescent compounds, luminescent compounds, ferromagnetic probes or radioactive compounds.

5. A kit for the diagnosis of tumors, comprising the monoclonal antibody of claim 1 or the same labelled antibody and other possible reagents conventionally used in immunodiagnostic methods.

### Claims for the following Contracting States : ES, GR

1. A method for the diagnosis of tumors in bioptic samples, characterized in that said samples are tested with a monoclonal antibody specific to the ED-B sequence of fibronectin from transformed cells, obtained from the hybridoma deposited at the European Collection of Animal Cell Cultures (ECACC) on April 21, 1988, under the No. 88042101, or with the same antibody, suitably labelled, for the presence of transformed fibronectin.

2. The method according to claim 1, which uses the monoclonal antibody labelled with enzymes, fluorescent compounds, luminescent compounds, ferromagnetic probes or radioactive compounds.

## Patentansprüche
### Patentansprüche für folgende Vertragsstaaten : AT, BE, CH, DE, FR, GB, IT, LI, LU, NL, SE

1. Monoclonaler Antikörper, der für die ED-B-Sequenz von Fibronectin von transformierten Zellen spezifisch ist, erhalten von dem bei der European Collection of Animal Cell Cultures (ECACC) am 21. April 1988 unter der Nummer 88042101 hinterlegten Hybridom

2. Hybridom ECACC 88042101.

3. Verfahren zur Diagnose von Tumoren in Biopsieproben, dadurch gekennzeichnet, daß diese Proben mit dem monoclonalan Antikörper nach Anspruch 1 oder mit den gleichen, in geeigneter Weise markierten Antikörper auf die Gegenwart von transformiertem Fibronectin getestet werden.

4. Verfahren nach Anspruch 3, wobei der monoclonale Antikörper markiert mit Enzymen, fluoreszierenden Verbindungen, lunineszierenden Verbindungen, ferromagnetischen Sonden oder radioaktiven Verbindungen verwendet wird.

**5.** Kit für die Diagnose von Tumoren, umfassend den monoclonalen Antikörper nach Anspruch 1 oder den gleichen markierten Antikörper und andere mögliche Reagenzien, die üblicherweise in immunodiagnostischen Methoden verwendet werden.

**Patentansprüche für folgende Vertragsstaaten : ES, GR**

**1.** Verfahren zur Diagnose von Tumoren in Biopsieproben, dadurch gekennzeichnet, daß die Proben mit einem monoclonalen Antikörper, der spezifisch für die ED-B-Sequenz von Fibronectin von transformierten Zellen ist, erhalten von dem bei der European Collection of Animal Cell Cultures (ECACC) am 21. April 1988 unter der Nummer 88042101 hinterlegten Hybridom, oder mit dem gleichen Antikörper, der in geeigneter Weise markiert ist, auf die Gegenwart von transformiertem Fibronectin getestet werden.

**2.** Verfahren nach Anspruch 1, wobei der monoclonale Antikörper markiert mit Enzymen, fluoreszierenden Verbindungen, lumineszierenden Verbindungen, ferromagnetischen Sonden oder radioaktiven Verbindungen verwendet wird.

**Revendications**
**Revendications pour les Etats contractants suivants : AT, BE, CH, DE, FR, GB, IT, LI, LU, NL, SE**

**1.** Anticorps monoclonal spécifique de la séquence ED-B de fibronectine provenant de cellules transformées, obtenu à partir de l'hybridome déposé à l'European Collection of Animal Cell Cultures (ECACC) le 21 Avril 1988, sous le N° 88042101.

**2.** Hybridome ECACC 88042101.

**3.** Méthode pour le diagnostic de tumeurs dans des échantillons prélevés par biopsie, caractérisée en ce que lesdits échantillons sont testés avec l'anticorps monoclonal selon la revendication 1 ou avec le même anticorps convenablement marqué, pour déterminer la présence de fibronectine transformée.

**4.** Méthode selon la revendication 3, laquelle utilise l'anticorps monoclonal marqué par des enzymes, des composés fluorescents, des composés luminescents, des sondes ferromagnétiques ou des composés radioactifs.

**5.** Kit pour le diagnostic de tumeurs, comprenant l'anticorps monoclonal selon la revendication 1 ou le même anticorps marqué et d'autres réactifs éventuels utilisés de façon classique dans des méthodes d'immunodiagnostic.

**Revendications pour les Etats contractants suivants : ES, GR**

**1.** Méthode pour le diagnostic de tumeurs dans des échantillons prélevés par biopsie, caractérisée en ce que lesdits échantillons sont testés avec un anticorps monoclonal spécifique de la séquence ED-B de fibronectine provenant de cellules transformées, obtenu à partir de l'hybridome déposé à l'European Collection of Animal Cell Cultures (ECACC) le 21 Avril 1988, sous le N° 88042101, ou avec le même anticorps convenablement marqué, pour déterminer la présence de fibronectine transformée.

**2.** Méthode selon la revendication 1, laquelle utilise l'anticorps monoclonal marqué par des enzymes, des composés fluorescents, des composés luminescents, des sondes ferromagnétiques ou des composés radioactifs.

FIG. 1

FIG. 2

10

FIG. 3

FIG. 4